# EUROPEAN PATENT SPECIFICATION

(11) **EP 3 085 233 B1**
(45) Date of publication and mention of the grant of the patent: **12.02.2020**
(21) Application number: 14835701.5
(22) Date of filing: 17.12.2014
(51) Int. Cl.: A01N 63/04, C12N 1/14, A01H 3/00

(54) **METHOD FOR INCREASING THE PRODUCTION OF FLOWERS, SEEDS, AND/OR FRUITS IN A PLANT**
VERFAHREN ZUR ERHÖHUNG DER PRODUKTION VON BLUMEN, SAMEN UND/ODER FRÜCHTEN EINER PFLANZE
PROCÉDÉ POUR AUGMENTER LA PRODUCTION DE FLEURS, GRAINES ET/OU FRUITS D'UNE PLANTE

(30) Priority: 17.12.2013 ES 201331839
(43) Date of publication of application: 26.10.2016
(73) Proprietor: Universidad Politécnica de Madrid, 28040 Madrid (ES); PlantResponse Biotech, S.L., 28223 Pozuelo de Alarcón (ES)
(72) Inventor: RAMÍREZ ZAPATA, Diana, E-28040 Madrid (ES); SACRISTÁN BENAYAS, María Soledad, E-28040 Madrid (ES); CUEVA GONZALEZ, Evelin Elizeth, E-28040 Madrid (ES); ALONSO GONZALEZ, Angela, E-28040 Madrid (ES); BORJA Y TOME, Marise, E-28223 Madrid (ES); ANTÓN RODRÍGUEZ, Diego, E-28223 Madrid (ES); PÉREZ JIMÉNEZ, Rosa María, E-28223 Madrid (ES)
(74) Representative: HGF Limited
(86) International application number: PCT/ES2014/070930
(87) International publication number: WO 2015/092104

(56) References cited:
- ES-A1- 2 439 393
- GANG TAO ET AL: "Endophytic Colletotrichum species from Bletilla ochracea (Orchidaceae), with descriptions of seven new speices", FUNGAL DIVERSITY, vol. 61, no. 1, 1 July 2013 (2013-07-01), pages 139-164, XP055114742, ISSN: 1560-2745, DOI: 10.1007/s13225-013-0254-5
- P.W P.F U J.H.C Crous Cannon Damm Woudenberg ET AL: "Colletotrichum species with curved conidia from herbaceous hosts", Fungal Diversity, 1 January 2009 (2009-01-01), page 45, XP055114671, Retrieved from the Internet: URL:http://library.wur.nl/WebQuery/wurpubs /387803 [retrieved on 2014-04-25] cited in the application
- REDMAN R S ET AL: "CONVERSION OF THE PATHOGENIC FUNGUS COLLETOTRICHUM MAGNA TO A NONPATHOGENIC, ENDOPHYTIC MUTUALIST BY GENE DISRUPTION", MOLECULAR PLANT-MICROBE INTERACTIONS, APS PRESS, vol. 12, no. 11, 1 November 1999 (1999-11-01), pages 969-975, XP009034000, ISSN: 0894-0282
- R. RODRIGUEZ ET AL: "More than 400 million years of evolution and some plants still can't make it on their own: plant stress tolerance via fungal symbiosis", JOURNAL OF EXPERIMENTAL BOTANY, vol. 59, no. 5, 16 February 2008 (2008-02-16), pages 1109-1114, XP055114681, ISSN: 0022-0957, DOI: 10.1093/jxb/erm342

## Description

### Field of the Invention

The field of the present invention is the agronomy sector, in particular, methods for increasing the production of flowers, seeds and/or fruits using the microorganism *Colletotrichum tofieldiae.*

### Background of the Invention

Plants in nature form symbiotic associations with microorganisms called mutualistic that confer benefits on their growth, survival and multiplication. These microorganisms can be isolated, and their beneficial properties used in crops to improve yield.

Genus *Colletotrichum* (Ascomycota, teleomorpho Glomerella) comprises over 60 species and species complexes. Morphologically it is characterized by having typically acervuli conidiomata, which can have bristles or not, with unicellular hyaline conidia that can be straight or curved, with a size preferably larger than 12 µ, typically granular. Conidia may also be formed from the mycelium or other conidia (microcyclic conidia). Conidia form appressoria when they germinate. Some species form stroma or sclerotia.
Genus *Colletotrichum* comprises species that are important pathogens of crops and, therefore, are the best known and best studied species. However, there are many species within the genus that have been cited as endophytes or epiphytes that cause no harm to the host plant (called commensals) or which may even be beneficial for the plant (mutualistic). Hyde et al. (Fungal Diversity 39 (2009) 147-182) present a complete description of all the species of the genus *Colletotrichum* currently known, with the list of hosts of these species cited in the literature and specifying the type of interactions established with each host (pathogenic, commensal or mutualistic). The evidence cited in this publication indicates that, within species or species complexes considered pathogenic, asymptomatic strains may exist. There are also cases of strains that can behave as pathogenic, commensal or mutualistic depending on the host in which they are inoculated. An example of this case is *C. orbiculare,* which behaves as a pathogen in cucurbits but may behave as mutualistic in tomato, conferring resistance to pathogens and drought and promoting vegetative plant growth when inoculated through the root.

### Description of the Invention

A preferred embodiment of the present invention is a method for increasing the production of flowers, seeds and/or fruits in a plant, characterized in that it comprises the step of contacting said plant with a composition comprising the microorganism *Colletotrichum tofieldiae* and/or extracts of said microorganism and/or filtrates of said microorganism, hereinafter method of the invention.
Hereinafter the term "microorganism of the invention" refers to the microorganism of the species *Colletotrichum tofieldiae.*

Increasing the production of flowers, seeds and/or fruits in a plant treated in accordance with the method of the invention is understood as the increase of number, size and/or weight of flowers, seeds and/or fruits of that plant against a plant that has not been treated in accordance with the method of the invention.

The species *Colletotrichum tofieldiae* is described in Damm et al. (Fungal Diversity 39 (2009) 45-87). This species is characterized by curved conidia and acervuli with bristles that can be dark brown or black. Conidia and bristles can form directly on the hyphae. The spores germinate to form appressoria of varying morphology and coloration. The definition of the species is also due to molecular characteristics regarding sequences of the 5.8S ribosomal subunit with the two spacer flanking regions (ITS), a 200-bp intron of the glyceraldehyde-3-phosphate dehydrogenase (GAPDH) gene, a partial sequence of the actin (ACT) gene, the chitin synthase 1 (CHS-1) gene, the beta-tubulin (tub2) gene and the histone 3 (HIS3) gene. The standard isolates used to describe the species *C*. *tofieldiae* come from *Tofieldia* spp. (monocotyledonous), *Lupinus polyphyllus* and *Dianthus* sp. (both dicotyledonous), so this species is able to colonize different species of hosts both mono and dicotyledonous. *C. tofieldiae* has not been cited as pathogenic or mutualistic of any host.

In the present invention, new *Colletotrichum tofieldiae* isolates have been isolated, identified and characterized which surprisingly have the ability to significantly increase the production of flowers, seeds and/or fruits in plants. Isolates of other fungal species of the same area did not show this ability. Plants increase the production of flowers, seeds and/or fruits as a consequence of the contact of any part of the plant with the microorganism *Colletotrichum tofieldiae* to establish a lasting relationship that induces the identified production increase. The present invention shows that the microorganism *C. tofieldiae* is translocated or dispersed to the various organs of the treated plant, and can remain there at least four months after treatment. This feature causes the effect of the microorganism on the plant to be more durable, lasting during the cycle of the plant and improving yield at the end thereof. This feature makes this microorganism more effective than other microorganisms used for similar purposes.

The method of the invention improves the production of flowers, fruits and seeds in a greater proportion than the increase of the vegetative growth of the plant. That is, the plant uses resources more efficiently, producing more and/or larger flowers, fruits and seeds in relation to the overall size of the plant. It is the first time that this feature is described in *Colletotrichum tofieldiae* strains of the invention. This is a phenomenon general to the *Colletotrichum tofieldiae* species because similar effects have been obtained with different strains of this species.

The method of the invention produces an increase in size and/or weight and/or number of flowers and/or fruits and/or seeds that can also be due to the better health or viability of the crop (for instance due to the reduction in the application of irrigation, fertilizers and/or pesticides, increased systemic resistance or increased herbicide resistance) and/or to increased seed germination and/or biocontrol characteristics such as a decrease in diseases due to the decrease in sensitivity to pathogens and/or improvement of previous infections.

The method of the invention produces at least one of the following effects:
- an increase in the overall yield of the plant;
- an increase in the quality of plant material;
- an increase in the number and/or size of flowers;
- an increase in the number, size and/or weight of fruits;
- an increase in the number, size and/or weight of seeds.

An increase in the overall yield preferably indicates an increase in the yield of harvestable parts of the plant. The increase in yield can be, for example, any increase such as an increase in the number of harvestable parts and/or an increase in weight gain and/or an increase in content of storage substances, interesting metabolites, etc. An increase in plant yield means that the harvestable parts of the plant are at least 2 %, preferably at least 25 %, more preferably 70 % and particularly preferred at least 500 % higher when the plant is contacted with the composition comprising spores, mycelium and/or any other part of the microorganism of the invention, the culture medium or the filtrate according to the invention compared to untreated plants.

An increase in quality preferably indicates an improvement of the desired qualities of a plant. This increase in quality may differ from plant to plant. With respect to ornamental plants, for example, Petunia, an increase in quality can mean an increase in the number of flowers or leaves. With respect to cereals, an increase in quality can mean an increase in the amount of protein or starch content in the seeds or a desired alteration in the structure or composition of storage substances.

In the method of the invention, a plant is contacted with a composition comprising the microorganism *Colletotrichum tofieldiae,* the culture medium or the filtrate according to the invention. The composition can be applied on the whole plant or on any part thereof, such as on the leaves, on the buds, on the flowers, on the fruits, on the cobs, on seeds, on bulbs, on tubers, on roots and on seedlings. The application of the composition to the plant can be performed at any stage, and as an example it can be applied to the seed before planting, at planting, after planting and before or after emergence, during the growing period and during the cultivation in seedbed, or at the time of transplantation of seedlings, or at the time of cutting or cuttings rooting, or at the time of growth in a plantation, or even in the reproductive period before flowering or during flowering or during fruits ripening process.

Thus, another embodiment is the method of the invention, wherein said composition is applied to the seeds of said plant.

Another embodiment is the method of the invention, wherein said composition is applied to the aerial parts of said plant.

Another embodiment is the method of the invention, wherein said composition is applied to the roots of said plant or to other underground parts of said plant.

The method of the invention includes treatment by spray or pulverization on whole plants or any part thereof of an appropriate dilution of the composition according to the invention or immersion of whole plants or any part thereof in said dilution. The method of the invention also includes treatment by dry dusting of whole plants or any part thereof of a composition according to the invention. The method of the invention includes pelleting, or coating seeds with a thin film of a composition according to the invention. The composition according to the invention can also be mixed with the irrigation fluid. The method of the invention also includes treatment by agar fragments with mycelium that are contacted with a part of the plant, either root, stem or leaves or even on the surface of the soil near the roots of the crop.

In the present specification, "filtrate" means a liquid culture medium obtained from the culture of the microorganism of the invention. It is possible to obtain a liquid culture medium free or essentially free of the microorganism of the invention. This culture medium can be prepared by first culturing the microorganism of the invention in a liquid culture medium and then separating the liquid culture medium from the microorganism of the invention. Separation can be carried out by different methods known to the person skilled in the art, for example, by centrifugation or filtration. It is possible, for example, to heat the medium with the microorganism of the invention twice up to about 80 °C for 30 min and then to remove the fungal material by centrifugation.

Preferably, the filtrate is obtained by filtration of the culture medium through a filter with a pore size of no more than 2 µm, preferably through a filter having a pore size of no more than 0.2 µm. The filtration step allows extraction of substantially all of the hyphae of the microorganism of the invention, more preferably filtration would also eliminate the spores and even more preferably this should eliminate any fungal material.

The present invention is also related to plants that, according to the method of the invention, have been contacted with a composition comprising the microorganism *Colletotrichum tofieldiae* and/or extracts of said microorganism and/or filtrates of said microorganism, and to the products produced from the harvested parts of said plants.

Another embodiment is the method of the invention, wherein said microorganism is a *Colletotrichum tofieldiae* strain deposited under deposit numbers CECT 20833, CECT 20834, CECT 20835 or CECT 20836.

The *Colletotrichum tofieldiae* strains have been deposited on 30/05/2013 (strains with deposit numbers CECT 20833, CECT 20834 and CECT 20835) and on 7/05/2013 (strain with deposit number CECT 20836) at the international depositary authority Spanish Type Culture Collection (CECT) with address at Parc Cientific Universitat de València, Catedrático Agustín Escardino, 9. 46980, Paterna, (Valencia), Spain, by Universidad Politécnica de Madrid, with address at c/ Ramiro de Maeztu, 7, 28040 Madrid, Spain.

The microorganism of the invention can be cultured on a wide variety of natural or synthetic substrates. For example, it can be cultured in different types of solid or liquid culture media, such as Potato/Dextrose/Agar (PDA) or Broth (PDB) media, and it can be propagated by techniques per se known by experts. It can also grow on various natural sources, such as leaves of various plants, pollen grains, oat flour, potato, carrot and cellulose. It can also grow on artificial sources such as paper or cardboard and polymers.

The culture medium can be constantly agitated during culture, for example with approximately 1 rps. Moreover, culture temperature can be set in the range between 20 and 30 °C.

Another embodiment is the method of the invention, wherein said microorganism is in the form of spores, hyphae, mycelium or sclerotia.

Another embodiment is the method of the invention, wherein said composition is applied to the substrate for the cultivation of said plant.

The substrate is preferably treated such that the microorganism of the invention is cultured in it before the substrate is used for cultivation of plants. Examples of treatment of the substrate include perfusion of a liquid into the substrate (by irrigation, injection or dripping), spraying, dusting or direct mix with the substrate. The method of the invention also comprises treatment of a hydroponic medium for hydroponic cultivation. The method of the invention comprises treatment of the substrate or hydroponic medium with a composition comprising the appropriate concentration of mycelium and/or spores and/or any other part of the microorganism of the invention, the culture medium or the filtrate according to the invention in liquid and/or solid form as granules or powder form.

The method of the invention can be carried out with a composition including the microorganism of the invention alone or formulated with inert ingredients. Examples of inert ingredients include fine powders or granules as minerals, such as clays, bentonite, calcite, diatomaceous and organic materials such as maize powder or nut skin powder, synthetic organic materials such as urea, salts such as calcium carbonate and ammonium sulfate, synthetic inorganic materials such as silicon oxide; or liquid diluents such as aromatic hydrocarbons such as xylene, alkyl benzene, methyl naphthalene, alcohols such as 2-5 propanol, ethylene glycol, propylene glycol, and ethylene glycol mono ethyl ether, ketones such as acetone, cyclohexanone and isophorone, vegetable oils such as soybean oil or cottonseed oil, aliphatic hydrocarbons derived from petroleum, esters, dimethyl sulfoxide, acetonitrile and water. Examples of surfactants include anionic surfactants such as salts of alkyl sulfate esters, alkyl aryl sulfonate salts, dialkyl sulfosuccinate salts, polyoxyethylene alkyl aryl phosphate ether ester salts, lignosulfonate salts and formaldehyde polycondensates, and nonionic surfactants such as polyoxyethylene alkyl aryl ethers, alkyl polyoxypropylene block copolymers, polyoxyethylene and fatty acid esters, and cationic surfactants such as alkyl trimethyl ammonium salts. Examples of other formulation auxiliary agents include water-soluble polymers such as polyvinyl alcohol or polyvinylpyrrolidone, polysaccharides such as agar, acacia, alginic acid and its salts, carboxy methyl cellulose, xanthan gum, inorganic materials such as aluminum and magnesium silicate, preservatives, coloring agents, stabilizers such as isopropyl acid phosphate and BHT.

Thus, another embodiment is the method of the invention, wherein said composition comprises minerals, organic materials, organic compounds, inorganic compounds, liquid diluents, alcohols, ketones, vegetable oils, aliphatic hydrocarbons, esters, dimethyl sulfoxide, acetonitrile, water, anionic surfactants, nonionic surfactants, cationic surfactants, water-soluble polymers, polysaccharides, preservatives, coloring agents, and/or stabilizers. Particularly, said minerals are selected from the group consisting of clays, bentonite, calcite and diatomaceous, said organic materials are selected from the group consisting of maize powder or nut skin powder, said organic compound is urea, said inorganic compounds are selected from the group consisting of calcium carbonate, ammonium sulfate, silicon oxide, aluminum and magnesium silicate, said liquid diluents are selected from the group consisting of aromatic hydrocarbons, aliphatic hydrocarbons, alcohols, ketones, vegetable oils, esters, dimethyl sulfoxide, acetonitrile and water, said anionic surfactants are selected from the group consisting of salts of alkyl sulfate esters, alkyl aryl sulfonate salts, dialkyl sulfosuccinate salts, polyoxyethylene alkyl aryl phosphate ether ester salts, lignosulfonate salts and formaldehyde polycondensates, said nonionic surfactants are selected from the group consisting of polyoxyethylene alkyl aryl ethers, alkyl polyoxypropylene block copolymers, polyoxyethylene and fatty acid esters, said cationic surfactants are alkyl trimethyl ammonium salts, said water-soluble polymers are polyvinyl alcohol or polyvinylpyrrolidone, said polysaccharides are selected from the group consisting of agar, acacia, alginic acid, alginic acid salts, carboxy methyl cellulose and xanthan gum and said stabilizers are isopropyl acid phosphate or BHT. Particularly, said aromatic hydrocarbons are selected from the group consisting of xylene, alkyl benzene, methyl naphthalene, said alcohols are selected from the group consisting of 2-5 propanol, ethylene glycol, propylene glycol and ethylene glycol mono ethyl ether, said ketones are selected from the group consisting of acetone, cyclohexanone and isophorone and said vegetable oils are soybean oil or cottonseed oil.

Another embodiment is the method of the invention, wherein said composition comprises fertigation salts, fertilizers, insecticides, nematocides, fungicides, bactericides and/or herbicides.

Another embodiment is the method of the invention, wherein said composition is a liquid, a solid, a paste or a gel.

Another embodiment is the method of the invention, wherein said composition is a powder, pill, tablet, granulate or emulsifiable concentrate.

Another embodiment is the method of the invention, wherein said composition is applied by spray, pulverization, immersion, irrigation or dusting.

Another embodiment is the method of the invention, wherein said plant is selected from the group consisting of gymnosperms, monocotyledonous and dicotyledonous. Particularly, said plant is a plant of the *Brassicaceae* family.

Another embodiment is the method of the invention, wherein said plant is a monocotyledonous plant.

Another embodiment is the method of the invention, wherein said plant is a barley plant *(Hordeum vulgare).*

Another embodiment is the method of the invention, wherein said plant is a maize plant (*Zea mays).*

In the present invention, the plant can be a natural or transgenic plant.

The method of the invention can be carried out with all possible plants and cells or tissues derived from such plants. In particular, it can be carried out with all the Gymnosperms and Angiosperms (monocotyledonous or dicotyledonous) or plant material derived from such plants, including trees, shrubs, herbs and grasses. Preferably, uses and processes are applicable to be used in useful plants or materials or cells of such plants, more preferably for agriculture, horticulture, forestry plants, ornamental plants, or of any other commercial interest. Among others, for illustrative purposes and without limiting the scope of the present invention: rice, wheat, barley, maize, soybean, rapeseed, tomato, bean, and different fruits (orange, lemon, etc.) and ornamental species.

Another embodiment of the invention is a propagation material of a plant comprising the microorganism *Colletotrichum tofieldiae* and/or extracts of said microorganism and/or filtrates of said microorganism. Particularly, said propagation material is selected from the group consisting of seeds, seedlings, young plants, cuttings, bulbs and tubers.

In the present specification "propagation material" means any type of cellular material from which a plant can germinate or grow. Examples of propagation material include, but without limitation: seeds, cuttings, cell suspensions, callus culture, tissue culture, protocorms, explants or germplasm. The propagation material can have different origins. For example, it can be freshly harvested, or derived from a stock, as a sample of seeds or a stock of frozen cells.

Another embodiment of the invention is a substrate for growing plants comprising the microorganism *Colletotrichum tofieldiae* and/or extracts of said microorganism and/or filtrates of said microorganism.

The substrate for growing plants may comprise the spore, the mycelium or any other part of the microorganism of the invention or the culture medium or filtrate thereof or any of the possible combinations of some of these components. The substrate may be liquid or solid.

Examples of substrates for the cultivation of plants are solidified natural or synthetic media and for the cultivation of the plants, especially *in vitro.* Other examples are soil, sand, peat or humus or mixtures thereof.

Another embodiment of the invention is a strain of the microorganism *Colletotrichum tofieldiae,* deposited under deposit numbers CECT 20833, CECT 20834, CECT 20835 and CECT 20836.

Another embodiment of the invention is the use of the microorganism *Colletotrichum tofieldiae* and/or extracts of said microorganism and/or filtrates of said microorganism for increasing the production of flowers, seeds and/or fruits in a plant.

Another embodiment of the invention is the use of the microorganism *Colletotrichum tofieldiae* and/or extracts of said microorganism and/or filtrate media of said microorganism for increasing the production of chlorophyll in a plant.

Another embodiment of the invention is the use of the microorganism *Colletotrichum tofieldiae* and/or extracts of said microorganism and/or filtrate media of said microorganism for increasing the production of flowers, seeds and/or fruits in a plant that is subject to drought or limited irrigation conditions.

Another embodiment of the invention is the primers identified by the sequences SEQ ID NO: 1 and 2.

### Brief Description of the Figures

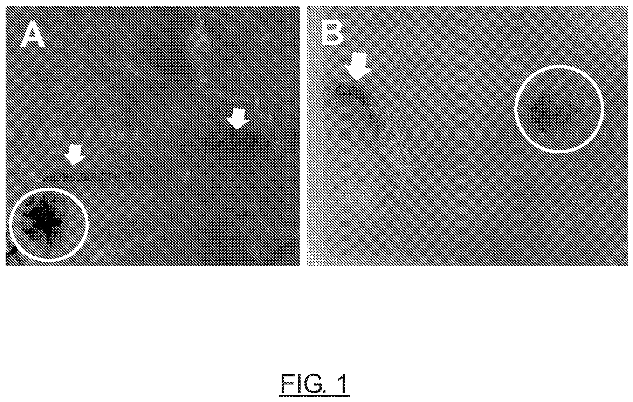

Figure 1. Re-isolation of *C. tofieldiae* from uninoculated organs of *A. thaliana* plants 60 days post inoculation. A. Isolate CECT 20834 re-isolated from leaf (circle) and stems (arrows). B. Isolate CECT 20833 re-isolated from root (arrow) and neck (circle).

### Preferred Embodiments

### Example 1: Screening of fungi that increase the productivity of the plants

*Arabidopsis thaliana* plants growing in natural conditions were harvested. These plants had no disease symptoms such as chlorosis, leaf spots and other types of lesions induced by pathogens. Fragments from the leaves of the rosette of the plants harvested were disinfected by dipping them in 20 % commercial bleach (1 % active chlorine) and gently stirring for 5 minutes. Then the fragments were rinsed twice in sterile water and placed in a moist chamber at room temperature (20 - 24 °C).

The fragments were regularly inspected, isolating the emerging mycelium in petri dishes with potato dextrose agar medium (PDA) with 200 mg/l of chloramphenicol. Among the isolates obtained, screening was performed to find fungi that are beneficial by resulting in increased seed production of the plant (Table 1). To perform the screening tests, *Arabidopsis thaliana* seeds accession Col-0 superficially sterilized were seeded in sterile substrate (peat and vermiculite at a 3:1 ratio). They were stratified at 4 °C for 3 days and then kept in a controlled growth chamber at 23 °C. The plants were treated at 3 weeks old (4-6 leaves) applying a piece of mycelium from any of the 3 mm diameter isolates on a leaf. The mycelium was obtained by subculturing the fungal mycelium obtained from an *Arabidopsis thaliana* wild plant without disease symptoms, on PDA plates.

The control plants were not subject to any treatment and were placed randomly among the treated plants. When the dehiscence of the leaf scapes started, these were covered with paper bags to collect the seeds. When the plants were dried, the seeds were harvested and weighed. The rest of the aerial part of the plant was also weighed. Among all those analyzed, we selected the isolate CECT 20833 of the genus *Colletotrichum,* since the plants treated with this isolate significantly increased (P<0.04) their seed production, the weight of the seeds of the treated plants being three times higher than that of the untreated plants (Table 1). The weight of the rest of the plant was not significantly different (P>0.05, Table 1).

**Table 1. Weights (mean ± standard error) of the seeds and dry weight of the rest of the plant of the treated or untreated plants with mycelium as described in Example 1. Different letters indicate significant differences between the treated plants and the control plants (P<0.05) according to ANOVA. The plants treated with the isolate CECT 20833 of the genus Colletotrichum (bold) had a significantly higher seed weight than the control plants, while the dry weight of the rest of the plant was not significantly higher.**

| TREATMENT | Control plants Weight of seeds per plant (mg) | Dry weight rest of the plant (mg) | Treated plants Weight of seeds per plant (mg) | Dry weight rest of the plant (mg) |
|---|---|---|---|---|
| *Alternaria* sp. | 28.0 ± 2.9^{a} | 177.9 ± 12.1^{a} | 21.6 ± 2.2^{b} | 151.1 ± 5.8^{b} |
| ***Colletotrichum sp.* CECT 20833** | **2.8 ± 1.1^{a}** | **85.1 ± 7.7** | **8.8 ± 1.7^{b}** | **90.7 ± 4.1** |
| *Plectosphaerella* sp. | 30.3 ± 5.8 | 218.9 ± 17.2 | 27.8 ± 4.1 | 233.6 ± 12.1 |
| *Trichoderma sp.* | 14.9 ± 1.6 | 143.8 ± 10.1^{a} | 16.5 ± 1.5 | 184.8 ± 11.6^{b} |
| *Trichoderma sp.* | 15.2 ± 1.8 | 101.5 ± 11.7 | 16.2 ± 1.6 | 126.3 ± 10.2 |
| *Ulocladium sp.* | 20.3 ± 3.1 | 169.7 ± 10.4 | 19.6 ± 1.8 | 166.0 ± 72 |

### Example 2: Identification of isolates of Colletotrichum sp. from Arabidopsis thaliana wild plants as Colletotrichum tofieldiae.

The isolate CECT 20833 of the genus *Colletotrichum* from the screening of Example 1 had dark mycelium and hyaline curved conidia in acervulus with dark bristles. Other isolates obtained in the same manner, identified as CECT 20834, CECT 20835, and CECT 20836, displayed similar morphology. All these isolates were identified as *Colletotrichum tofieldiae* according to the morphology and the sequences of several loci described in Damm et al. (Fungal Diversity 39 (2009) 45-87) which were compared with sequences of standard isolates deposited in the database accessible in http://www.cbs.knaw.nl/Colletotrichum/, housed at the Fungal Biodiversity Centre (CBS, Centraalbureau voor Schimmelcultures).

The morphology of these isolates coincides with that described in Damm et al. (Fungal Diversity 39 (2009) 45-87) for *C. tofieldiae,* adding that culture in PDA (potato dextrose agar) medium or equivalent, or on filter paper or on *A. thaliana* leaves produces very dark colonies, that the mycelium in culture darkens and hardens over time and that it may have thickened structures of chlamydospores type and/or microsclerotia type.

The sequence similarities (number of identical nucleotides/number of overlapping nucleotides) of the sequences compared between the isolates CECT 20833, CECT 20834, CECT 20835 and CECT 20836 and the standard isolates CBS 168.49, CBS 495. 85 and IMI 288810 of the Fungal Biodiversity Centre, used in Damm et al. (Fungal Diversity 39 (2009) 45-87) to define the species *C*. *tofieldiae,* were higher than 92 %. The compared sequences were those of the 5.8S ribosomal subunit with the two spacer flanking regions (ITS, similarity > 95 %), a 200-bp intron of the glyceraldehyde-3-phosphate dehydrogenase gene (GAPDH, similarity >92 %), a partial sequence of the actin gene (ACT, similarity >99 %), and the histone 3 gene (HIS3, similarity > 98 %). These comparisons were performed with the paired sequence alignments tool MycoID housed at the MycoBank website (www.mycobank.org) of the International Mycological Association.

The set of these characteristics define the species of *Colletotrichum* that correspond to the microorganism of the present invention.

### Example 3: Colletotrichum tofieldiae isolates persisted in treated plants at least two months post inoculation and translocated or dispersed to other organs different from the inoculated leaf such as root, crown, other leaves and stems.

*Arabidopsis thaliana* seeds accession Col-0 superficially sterilized were seeded in sterile substrate (peat and vermiculite at a 3:1 ratio). They were stratified at 4 °C for 3 days and then kept in a controlled growth chamber at 23 °C, in short cycle (10 hours of light per day). The plants were treated at 3 weeks old with the *Colletotrichum tofieldiae* isolates CECT 20833, CECT 20834 and CECT 20835 by the application to three leaves of a drop of a suspension of spores at 10⁶ spores/ml. The whole plants were harvested 60 days post inoculation (dpi).

Roots, leaves from the rosette and stems were removed and cut into fragments of approximately 25 mm², disinfected as described previously and incubated in a humid chamber at room temperature. After one week, *Colletotrichum tofieldiae* was re-isolated from at least one sample of all the inoculated plants. The fungus was re-isolated from leaves other than the inoculated ones, from the crown, from the roots and from the stem (Table 2, Figure 1). No *C. tofieldiae* growth was observed in any of the control plants treated with distilled water.

**Table 2. Percentage of plants from which C. tofilediae was re-isolated from organs other than those inoculated (leaves, stems, neck or root) 60 days after the treatment. ¹ Percentage of plants from which C. tofilediae was re-isolated in at least one sample of an uninoculated organ.**

| | Re-isolation percentage | | | | |
|---|---|---|---|---|---|
| Isolate | Leaves | Stem | Neck | Root | Total¹ |
| CECT 20833 | 80 % | 0 % | 67 % | 33 % | 100 % |
| CECT 20834 | 100 % | 17 % | 50 % | 83 % | 100 % |
| CECT 20835 | 100 % | 8 % | 75 % | 67 % | 100 % |

### Example 4: The Colletotrichum tofieldiae isolate CECT 20834 increased the production of seeds and the weight of seeds per plant in plants treated with mycelium or spores

*Arabidopsis thaliana* seeds accession Col-0 superficially sterilized were seeded in sterile substrate (peat and vermiculite at a 3:1 ratio), stratified at 4 °C for 3 days and then kept in a controlled growth chamber at 23 °C. At three weeks old, they were subjected to one of the following treatments a) Treatment with a piece of mycelium from isolate CECT 20834 in the same manner as in example 1; b) Application of a suspension of spores from isolate CECT 20834 in sterile distilled water at a concentration of 10⁶ spores/ml applied by spray over the whole plant and the substrate under the same; c) Control treatment consisting of the application of sterile distilled water by spray over the whole plant and the substrate under the same.

In the case of treatment b, the spores were obtained resuspending in sterile distilled water the spores obtained from the fungus culture in PDA medium. The plants subjected to each of the treatments were arranged in a randomized manner. When starting the dehiscence of the leaf scapes, these were covered with paper bags to collect the seeds. When the plants were dried, the seeds were collected and weighed. The rest of the aerial part of the plant was also weighed.

The seed production per plant was measured as the weight of all the seeds harvested from each plant at the end of the cycle. The seed production of the plants treated with CECT 20834 was significantly higher than that of the control plants (P<0.001), with an increase in weight of 37 % for the plants treated with mycelium and of 56 % for the plants treated with the spray suspension of spores (Table 2). The increase in seed production is partly due to the production of heavier seeds, since the weight of 100 seeds of the plants treated with CECT 20834 showed a significant increase (P<0.06) of up to 42 % with respect to the control plants. The dry weight of the rest of the plant was not significantly different (P>0.10). The fungus was re-isolated, 6 weeks after the treatment, from 75 % of the plants inoculated with mycelium and from 100 % of the plants inoculated with spray. This example shows that the ability to increase the production of seeds is not exclusive to isolate CECT 20833, but it can be extended to other *C. tofieldiae* isolates and that the treatment with spores produces similar results to treatment with mycelium.

**Table 3. Weights (mean ± standard error) of the seeds per plant and of 100 seeds per each plant and dry weight of the rest of the plant (plant without seeds) of the plants treated with mycelium or spore suspension of isolate CECT 20834 or distilled water spray as described in Example 4. *Different letters show significant differences according to ANOVA (P<0.001, for the weight of seeds per plant and P<0.06 for the weight of 100 seeds). No significant differences were found in the weight of the plant without seeds (P>0.10). The increase in percentage, comparing treated and untreated plants, is shown in brackets.**

| TREATMENT | Weight of seeds per plant (mg)* | Weight of 100 seeds per plant (mg)* | Dry weight of the plant without seeds (mg)* |
|---|---|---|---|
| Spray with distilled water | 35.0 ± 3.6^{a} | 1.17 ± 0.11^{a} | 213.9 ± 12.0 |
| Mycelium | 47.5 ± 3.7^{b} (37 %) | 1.66 ± 0.13^{b} (42 %) | 249.3 ± 15.3 (16 %) |
| Spray with spore suspension | 56.1 ± 2.9^{b} (56 %) | 1.65 ± 0.19^{b} (41 %) | 239.6 ± 16.9 (12 %) |

### Example 5: The Colletotrichum tofieldiae isolates CECT 20835 and CECT 20836 increase the production of seeds (seed weight per plant) in plants treated with a spore suspension applied by spray. The increase is proportional to the persistence of the fungus in the plant.

*Arabidopsis thaliana* seeds accession Col-0 superficially sterilized were seeded in sterile substrate (peat and vermiculite at a 3:1 ratio), stratified at 4 °C for 3 days and then kept in a controlled growth chamber at 23 °C. At three weeks old, they were subjected to any of the following treatments a) Application of a suspension of spores from *Colletotrichum* sp isolate CECT 20835; b) Application of a suspension of spores from *Colletotrichum* sp isolate CECT 20836; c) Control treatment consisting of the application of sterile distilled water. All the treatments were applied by spray throughout the plant and the soil under the same.

The inoculum of treatments a, b and c comes from spore suspensions kept in 30 % glycerol at -80°C and diluted in sterile distilled water at a concentration of 10⁶ spores/ml. After the application of the treatment, the plants were kept at 25 °C, short cycle. Two weeks after the treatment, the fungus was re-isolated from 17 % of the plants inoculated with CECT 20835, from 20 % of the plants inoculated with CECT 20836 and from no plant treated only with water. Six weeks after the treatment, the fungus was re-isolated from 60 % of the plants treated with CECT 20835 and from none of the plants treated with CECT 20836 or only water. When the dehiscence of the leaf scapes started, these were covered with paper bags to collect the seeds. When the plants were dried, the seeds were harvested and weighed. The rest of the aerial part of the plant was also weighed.

The seed production per plant was measured as the weight of all the seeds harvested from each plant at the end of the cycle. The ANOVA of the weights of the seeds indicates significant differences in the average weight of seeds per plant among plants subjected to the different treatments (P<0.0001, Table 4): The plants treated with CECT 20835 had the highest average weight of seeds per plant, with an increase of 550 % with respect to the plants treated only with water and the plants treated with CECT 20836 had an increase of average weight of seeds of 400 % with respect to the plants treated only with water. No significant differences were found in the weight of the plant without seeds (P>0.10). This example shows that the effect of applying *Colletotrichum tofieldiae* may be proportional to the persistence of the fungus in the plant.

| TREATMENT | Weight of seeds per plant (mg)* | Dry weight of the plant without seeds (mg) |
|---|---|---|
| Spray with distilled water | 5.7 ± 2.7^{a} | 273.6 ± 14.9 |
| Spray with spore suspension of CECT 20835 | 38.7 ± 2.5^{b} (550 %) | 266.2 ± 20.7 |
| Spray with spore suspension of CECT 20836 | 30. ± 2.7^{c} (400 %) | 232.9 ± 15.5 |

Table 4. Weights (mean ± standard error) of the seeds per plant and dry weight of the rest of the plant of the plants (plant without seeds) treated as described in Example 5. * Different letters show significant differences according to ANOVA among the different treatments (P<0.0001). The weight of the rest of the plant was not significantly different among the different treatments (P>0.10). The increase in percentage, compared between plants treated with the different isolates and the control plants, is shown in brackets.

### Example 6: Colletotrichum tofieldiae increased the number of fruits of the plants treated with a spore suspension both in plants seeded in sterile substrate and in plants seeded in non-sterile substrate.

*Arabidopsis thaliana* seeds accession Col-0 superficially sterilized were seeded in sterile and non-sterile substrate (peat and vermiculite at a 3:1 ratio). They were stratified at 4 °C for 3 days and then kept in a controlled growth chamber at 23 °C. At three weeks old, they were subjected to any of the following treatments a) Application of a suspension of spores from *C*. *tofieldiae* isolate CECT 20833; b) Application of a suspension of spores from *C*. *tofieldiae* isolate CECT 20835; c) application of sterile distilled water. All the treatments were applied by spray throughout the plant. The inoculum of treatments a and b comes from spores suspensions kept in 30 % glycerol at -80°C, washed and diluted in sterile distilled water at a concentration of 10⁶ spores/ml. The plants were randomly distributed. The number of fruits per plant was counted at the time of initiation of the opening of the siliques. The results are shown in Table 5. The plants treated with the *C*. *tofieldiae* isolates CECT 20833 and CECT 20835 showed a significant increase in fruits of up to 39 %. This experiment demonstrates that the increase in the production of seeds is due in part to the production of a greater number of fruits per plant. This experiment demonstrates that the effect of the fungus remains on plants on non-sterile substrate.

**Table 5. Number of fruits (mean ± standard error) per plant treated with a spore suspension from different C. tofieldiae isolates as described in Example 6. *Different letters show significant differences in ANOVA (P<0.08 for the sterile substrate and P<0.04 for the non-sterile substrate). The increase in percentage, compared between treated and untreated plants, is shown in brackets.**

| | No. of fruits* | |
|---|---|---|
| | Sterile substrate | Non-sterile substrate |
| Spray with distilled water | 53.3 ± 6.5^{a} | 11.3 ± 0.7^{a} |
| *C. tofieldiae* CECT 20833 | 73.9 ± 7.0^{b} (39 %) | 14.7 ± 1.4^{b} (31 %) |
| *C. tofieldiae* CECT 20835 | 60.4 ± 5.8^{b} (13 %) | 14.3 ± 0.8^{b} (27 %) |

### Example 7: Colletotrichum tofieldiae increased the number of fruits of the plants seeded in substrates (sterile or non-sterile) treated with a spore suspension.

*Arabidopsis thaliana* seeds accession Col-0 superficially sterilized were seeded in sterile and non-sterile substrate (peat and vermiculite at a 3:1 ratio). They were stratified at 4 °C for 3 days and then kept in a controlled growth chamber at 23 °C. When the plants were three weeks old 200 µl per plant of a) a spore suspension of *C. tofieldiae* isolate CECT 20833; b) a spore suspension of *C*. *tofieldiae* isolate CECT 20835; c) sterile distilled water, were deposited in the substrate, next to the plant. The inoculum of treatments a and b comes from spores suspensions kept in 30 % glycerol at -80°C washed and diluted in sterile distilled water at a concentration of 10⁶ spores/ml. The plants were randomly distributed. The number of fruits per plant was counted at the time of initiation of the opening of the siliques. The results are shown in Table 6. The plants treated with the *C*. *tofieldiae* isolates CECT 20833 and CECT 20835 show an increase in fruits of up to 27 %.

**Table 6. Number of fruits (mean ± standard error) per plant treated with a spore suspension of different Colletotrichum isolates applied directly to the substrate as described in Example 7. * The increase in percentage, compared between treated and untreated plants, is shown in brackets.**

| | No. of fruits* | |
|---|---|---|
| | Sterile substrate | Non-sterile substrate |
| Spray with distilled water | 53.8 ± 6.0 | 16.6 ± 1.5 |
| *C. tofieldiae* CECT 20833 | 64.8 ± 7.0 (20 %) | 17.9 ± 1.5 (8 %) |
| *C*. *tofieldiae* CECT 20835 | 68.4 ± 7.3 (27 %) | 17.6 ± 5.1 (6 %) |

### Example 8: Colletotrichum tofieldiae persisted in treated maize and rapeseed plants, at least 38 days post inoculation.

Maize seeds of the variety FAO700, and rapeseed seeds of the variety Westar superficially sterilized were seeded in sterile substrate (peat and vermiculite at a 3:1 ratio) and then kept in a controlled growth chamber at 23 °C, in short cycle (10 hours of light per day). The plants were treated at 3 weeks old with the *C. tofieldiae* isolate CECT 20833 by spray application of 120 ml of a spore suspension at 10⁶ spores/ml in the presence of 0,75 % surfactant (alkyl polysaccharide). The first and the second leaves of seven plants were harvested 23 dpi, while the third and the fourth leaves were collected 38 dpi.

The leaves were separated and cut into fragments of approximately 25 mm², disinfected as described previously and incubated in a humid chamber at room temperature. After one week, *C. tofieldiae* was re-isolated from at least 64,3 % of the samples collected 38 dps from seven maize inoculated plants and 7 % of the samples collected from seven rapeseed inoculated plants (Table 7). No *C. tofieldiae* growth was observed in any of the control plants treated with distilled water.

**Table 7. Number of maize or rapeseed leaves and percentage of those from which Colletotrichum tofilediae was re-isolated 23 (first and second leaves) and 38 days (third and fourth leaves) after treatment.**

| Crop | Days post spray | Sampled leaf (seven plants) | Number of maize leaves having acervuli (seven leaves) | Re-isolation percentage per leaf | Re-isolation percentage per leaf of the same plant |
|---|---|---|---|---|---|
| Maize | 23 | Leaf 1 | 1 | 14.3 % | 42.8 % |
| | | Leaf 2 | 5 | 71.4 % | |
| | 38 | Leaf 3 | 5 | 71.4 % | 64.3 % |
| | | Leaf 4 | 4 | 57.1 % | |
| Rapeseed | 23 | Leaf 1 | 7 | 100 % | 78.6 % |
| | | Leaf 2 | 4 | 57,14 % | |
| | 38 | Leaf 3 | 0 | 0 % | |
| | | Leaf 4 | 1 | 14,28 % | 7 % |

### Example 9: Colletotrichum tofieldiae can be inoculated by different methods in maize and barley, it is translocated from the seeds and roots to the leaves, and increases the chlorophyll content.

Washed maize seeds of the variety FAO700 and barley seeds of the variety Arturio were subjected to different treatments (36 plants per treatment):
1. Immersion treatment: the seeds were inoculated by immersion for 2 h in a beaten mycelium suspension of isolate CECT 20833, grown in PDB for 10 days, at an optical density of 1.2 and seeded in a substrate mixture of peat and vermiculite at a 3:1 ratio.
2. Rye treatment: the seeds were seeded in a substrate mixture of peat and vermiculite at a 3:1 ratio to which rye seeds were added (35 g/l substrate) that had been inoculated 8 days before with mycelium discs of isolate CECT 20833.
3. Peat treatment: the seeds were seeded in a substrate mixture of peat and vermiculite at a 3:1 ratio to which a mixture of peat and bran was added (20 g/l substrate) that had been inoculated two weeks before with spores of isolate CECT 20833. An amount of 1,5x10⁴ spores/gram of peat and bran mixture.
4. Spray treatment: the seeds were seeded in a substrate mixture of peat and vermiculite at a 3:1 ratio and at 3 weeks old were inoculated by spray application of 120 ml of a mycelium suspension of isolate CECT 20833, that had been grown in PDB for 10 days, adjusted at an optical density of 1.2, in the presence of 0,75 % surfactant (alkyl polysaccharide).
5. Irrigation treatment: the seeds were seeded in a substrate mixture of peat and vermiculite at a 3:1 ratio and inoculated by irrigation (5 ml) with a beaten mycelium suspension of isolate CECT 20833 that had been grown in PDB for 10 days, adjusted at an optical density of 1.2.

Once seeded, the plants were kept in a greenhouse at 23 °C, in short cycle (10 hours of light per day). After 25 days, the leaves were separated and cut into fragments of approximately 25 mm², disinfected as described previously and incubated in a humid chamber at room temperature. After one week, *C. tofieldiae* was re-isolated from all the rye treatments and from all the maize treatments except from the seed immersion (Table 8). No *C. tofieldiae* growth was observed in any of the control plants treated with distilled water. Moreover, after three months, the plants were sampled and leaf tissue was taken, which was kept for 18 hours in 80 % ethanol. After this time, the absorbance (OD₆₆₃) of the suspension was measured as an indicator of the chlorophyll a concentration. It was found that in the rye and peat treatments the absorbance was significantly higher than in the uninoculated control plants (Table 9).

**Table 8. Re-isolation percentage of isolate CECT 20833 from maize and barley plants after the different treatments and absorbance of maize samples OD₆₆₃ (chlorophyll a). *Different letters show significant differences in ANOVA (P<0.05) Example 10: Colletotrichum tofieldiae can be inoculated both as conidia and as mycelium both in the substrate and as spray in maize. It mobilizes from the root to the leaves and vice versa, increasing the chlorophylls content.**

| Treatment | *N* | Maize: re-isolation percentage | Barley: re-isolation percentage | Maize: absorbance OD₆₆₃ (chlorophyll a)* |
|---|---|---|---|---|
| Immersion control | 32 | 0 % | 0 % | - |
| Immersion | 32 | 0 % | 20 % | - |
| | | | | |
| Rye control | 32 | 0 % | 0 % | 0.24 ± 0.02^{a} |
| Rye | 32 | 90 % | 40 % | 0.33 ± 0.03^{b} |
| | | | | |
| Peat control | 32 | 0 % | 0 % | 0.15 ± 0.02^{a} |
| Peat | 32 | 80 % | 10 % | 0.25 ± 0.02^{b} |
| | | | | |
| Spray control | 32 | 0 % | 0 % | 0.42 ± 0.02^{a} |
| Spray | 32 | 30 % | 50 % | 0.37 ± 0.02^{a} |
| | | | | |
| Irrigation control | 32 | 0 % | 0 % | 0.17 ± 0.02^{a} |
| Irrigation | 32 | 70 % | 50 % | 0.16 ± 0.02^{a} |

Washed maize seeds of the variety FAO700 were subjected to different treatments (54 plants per treatment):
1. Peat-mycelium treatment: the seeds were seeded in a peat:vermiculite substrate (3:1) plus a mixture of peat and bran (20 g/l) inoculated with a beaten mycelium suspension of isolate CECT 20833 as described in example 9.
2. Peat-spores treatment: the seeds were seeded in a peat:vermiculite substrate (3:1) plus a mixture of peat and bran (20 g/1) inoculated two weeks before with a spore suspension at 10⁶ spores/ml of isolate CECT 20833.
3. Spray-spores treatment: the seeds were seeded in a peat:vermiculite substrate (3:1) and the plants were inoculated at 3 weeks old with the *C. tofieldiae* isolate CECT 20833 by spray application of 120 ml of a spore suspension at 10⁶ spores/ml in the presence of 0,5 % surfactant (alkyl polysaccharide).
4. Spray-mycelium treatment: the seeds were seeded in a peat:vermiculite substrate (3:1) and the plants were inoculated at 3 weeks old with the *C. tofieldiae* isolate CECT 20833 by spray application of 120 ml of a mycelium suspension at an optical density of 1.5 in the presence of 0,5 % surfactant (alkyl polysaccharide).

Once seeded, the plants were kept in a greenhouse at 23 °C, in short cycle (10 hours of light per day) for eight weeks. The leaves or the roots were separated and cut into fragments of approximately 25 mm², disinfected as described previously and incubated in a humid chamber at room temperature. After 30 days, *C. tofieldiae* was re-isolated from all the treatments (Table 9). No *C. tofieldiae* growth was observed in any of the control plants treated with a distilled water spray or seeded with peat. Moreover, the chlorophyll content index (CCI) was measured using the opti-sciences CCM 200 meter. It was observed that in all the treatments the inoculated plants had a CCI higher than the uninoculated control plants, except for the spray treatment spores (Table 9).

**Table 10. Re-isolation percentage in root and leaves of maize plants and chlorophyll content (CCI) (mean ± standard error) after the different treatments. *Different letters show significant differences in ANOVA (P<0.05).**

| Treatment | *N* | Root: re-isolation percentage | Leaf: re-isolation percentage | *N* (number of measurements) | CCI* |
|---|---|---|---|---|---|
| Peat mycelium control | 54 | 0 % | 0 % | 50 | 2.82 ± 0.09^{a} |
| Peat mycelium | 54 | 67 % | 20 % | 38 | 3.57 ± 0.12^{b} |
| Peat spores control | 54 | 0 % | 0 % | 56 | 3.27 ± 0.14^{a} |
| Peat spores | 54 | 67 % | 25 % | 34 | 3.78 ± 0.16^{b} |
| | | | | | |
| Mycelium spray control | 54 | 0 % | 0 % | 60 | 3.97 ± 0.13^{a} |
| Mycelium spray | 54 | 33 % | 45 % | 48 | 5.84 ± 0.44^{b} |
| Spores spray control | | | 0 % | 70 | 3.46 ± 0.10^{a} |
| Spores spray | 54 | 42 % | 35 % | 64 | 2.99 ± 0.10^{b} |

### Example 11: Colletotrichum tofieldiae increased the number of grains per cob and the weight per cob in maize plants treated with the isolate CECT 20833 under normal irrigation and limited irrigation conditions.

Washed maize seeds of the variety FAO700 were inoculated by immersion for 16 h in a spore suspension at 10⁶ spores/ml, seeded in substrate (peat and vermiculite at a 3:1 ratio) and kept in a controlled growth chamber at 23 °C. When the plants were three weeks old they were taken to the field, where they were transplanted to soil in four rows separated by 70 cm and with a distance of 25 cm between plants within each row. The control plants and the plants inoculated with C. *tofieldiae* were planted in randomized blocks within each row. After a month with normal irrigation (water 100 %), two of the rows were subjected to limited irrigation with 30 % irrigation with respect to the control plants. At the end of the growing season, the number of grains per cob and the weight of each cob were recorded. The results are shown in Table 10. The plants treated with C. *tofieldiae* showed an increase of grains of up to 47 % and of average weight per cob of up to 70 % when the plants were subjected to limited irrigation.

**Table 10. Average production of the number of grains (mean ± standard error) and weight per cob (mean ± standard error) of plants germinated from seeds treated with a C. tofieldiae spore suspension under normal irrigation and limited irrigation conditions. * The increase in the percentage compared between treated and untreated plants is shown in brackets. Different letters show significant differences in ANOVA (P<0.05)**

| Treatment | *N* | Number of grains per cob | *P-*value | Average weight per cob | *P-*value |
|---|---|---|---|---|---|
| Normal irrigation (100 %) | | | | | |
| Control | 19 | 439 ± 51 | - | 169 ± 19 | - |
| *C. tofieldiae* Inoculation | 28 | 450 ± 36 (2 %)* | 0.866 | 211± 17 (25 %) | 0.119 |

| Limited irrigation (30 %) | | | | | |
|---|---|---|---|---|---|
| Control | 17 | 267 ± 27 | - | 102± 24 | - |
| *C. tofieldiae* Inoculation | 20 | 393 ± 56 (47 %) | 0.063 | 173 ± 22 (70 %) | 0.034 * |

### Example 12: When Colletotrichum tofieldiae colonizes plants, it produces an increase in the number of grains per cob and in the weight of the cobs per maize plant treated with isolate CECT 20833.

Washed maize seeds of the variety FAO700 were seeded in the soil in four rows separated by 70 cm and with a distance of 25 cm between plants within each row. For the inoculation with *C. tofieldiae,* 200 ml/seed of peat vermiculite mixture was deposited in the bottom of the sowing furrow at a concentration of 10⁶ CFU/ml that had been inoculated two weeks before with spores with a concentration of 10⁶ spores/ml of isolate CECT 20833. The control plants and the plants inoculated with *C*. *tofieldiae* were seeded in randomized blocks within each row. At the end of the growing season, the number of grains per cob was recorded. Real-time PCR (RT PCR) of the tissue of brace roots of maize plants was performed in parallel with the primers CT_RTPCR L (SEQ ID NO: 1) and CT_RTPCR_R (SEQ ID NO: 2) specific for *C. tofieldiae.* The results are shown in Table 11. The plants colonized with *C. tofieldiae* show increased production of cobs of up to 43 % and increased production of seeds of up to 35 % when the plants are subjected to limited irrigation with 30 % irrigation with respect to the control plants.

**Table 11. Average production (cob weight per plant (mean ± standard error) and number of grains per plant (mean ± standard error)) of plants germinated from seeds treated with a C. tofieldiae spore suspension under conditions of normal irrigation. The increase of the percentage compared between colonized and non-colonized plants is shown in brackets. Different letters show significant differences in ANOVA (P<0.05)**

| Group | N | Average cob weight per plant | P-value | Average number of grains per plant | P-value |
|---|---|---|---|---|---|
| *Control* | 26 | 237 ± 19 | - | 574 ± 122 | - |
| *C. tofieldiae* | 7 | 339 ± 76 (42 %) | 0.063 | 775 ± 48 (35 %) | 0.079 |

## Claims

1. A method for increasing the production of flowers, seeds and/or fruits in a plant, comprising the step of contacting said plant with a composition, the composition comprising the microorganism *Colletotrichum tofieldiae* and/or filtrates of said microorganism.

2. Method according to claim 1, **characterized in that** said microorganism is a strain of *Colletotrichum tofieldiae* selected from deposit identification numbers CECT 20833, CECT 20834, CECT 20835 or CECT 20836.

3. Method according to any one of claims 1 or 2, **characterized in that** said microorganism is in the form of spores, hyphae, mycelium or sclerotia.

4. Method according to any one of claims 1 to 3, **characterized in that** said contacting comprises the aplication of the composition to seeds.

5. Method according to any one of claims 1 to 3, **characterized in that** said contacting comprises the aplication of the composition to aerial parts of the plant.

6. Method according to any one of claims 1 to 3, **characterized in that** said contacting comprises the aplication of the composition to roots, bulbs or tubers of the plant.

7. Method according to any one of claims 1 to 3, **characterized in that** said contacting comprises the aplication of the composition to a substrate able to cultivate said plant.

8. Method according to any one of claims 1 to 7, **characterized in that** said composition comprises minerals, organic materials, organic compounds, inorganic compounds, liquid diluents, alcohols, ketones, vegetable oils, aliphatic hydrocarbons, esters, dimethyl sulfoxide, acetonitrile, water, anionic surfactants, nonionic surfactants, cationic surfactants, water soluble polymers, polysaccharides, preservatives, coloring agents and / or stabilizing agents.

9. Method according to claim 8, **characterized in that** said minerals are selected from the group consisting of clay, bentonite, calcite and diatomaceous cob; the organic materials are selected from the group consisting of corn powder and nut skin powder; said organic compound is urea; said inorganic compounds are selected from the group consisting of calcium carbonate, ammonium sulfate, silicon oxide and magnesium aluminum silicate; said liquid diluents are selected from the group consisting of aromatic hydrocarbons, aliphatic hydrocarbons, alcohols, ketones, vegetable oils, esters, dimethyl sulfoxide, acetonitrile and water; said anionic surfactants are selected from the group consisting of salts of sulphate alkyl esters, alkylaryl sulfonate salts, dialkyl sulfosuccinate salts, salts of polyoxyethylene alkylaryl ether phosphate esters, polyoxyethylene lignosulfonate salts and formaldehyde polycondensates; said nonionic surfactants are selected from the group consisting of alkylaryl polyoxyethylene ethers, alkylpolyoxipropylene block copolymers and polyoxyethylene fatty acid esters; said cationic surfactants are alkyl trimethylammonium salts; said polymers are water soluble polyvinyl alcohol or polyvinylpyrrolidone; said polysaccharides are selected from the group consisting of agar, acacia, alginic acid, salts of alginic acid, carboxy methyl cellulose and xanthan gum; and said stabilizing agents are isopropyl phosphate acid or BHT.

10. Method according to claim 9, **characterized in that** said aromatic hydrocarbons are selected from the group consisting of xylene, alkyl benzene and methyl naphthalene; said alcohols are selected from the group consisting of 2-5 propanol, ethylene glycol, propylene glycol and ethylene glycol monoethyl ether; said ketones are selected from the group comprising acetone, cyclohexanone and isophorone; and said vegetable oils are soybean oil or cottonseed oil.

11. Method according to any one of claims 1 to 10, **characterized in that** said composition comprises substrate fertirrigation salts, fertilizers, insecticides, nematicides, fungicides, bactericides and / or herbicides.

12. Method according to any one of claims 1 to 11, **characterized in that** said composition is a liquid, a solid, a paste or a gel.

13. Method according to any one of claims 1 to 12, **characterized in that** said composition is a powder, pill, tablet, granule or an emulsifiable concentrate.

14. Method according to any one of claims 1 to 13, **characterized in that** said contacting comprises application by spray, pulverization, immersion, irrigation or dusting.

15. Method according to any one of claims 1 to 14, **characterized in that** said plant is a gymnosperms or a dicot.

16. Method according to any one of claims 1 to 14, **characterized in that** said plant is of the family *Brassicaceae.*

17. Method according to any one of claims 1 to 14, **characterized in that** said plant is a monocot.

18. Method according to any one of claims 1 to 14, **characterized in that** said plant is barley *(Hordeum vulgare).*

19. Method according to any one of claims 1 to 14, **characterized in that** said plant is corn (*Zea mays*).

20. Plant propagating material comprising a strain of *Colletotrichum tofieldiae* selected from deposit identification numbers CECT 20833, CECT 20834, CECT 20835 or CECT 20836.

21. Plant propagating material according to claim 20, in which said material is selected from the group consisting of seeds, seedlings, young plants, cuttings, bulbs and tubers.

22. Substrate for plant cultivation comprising the microorganism *Colletotrichum tofieldiae* and/or said microorganism filtrates.

23. Strain of the microorganism *Colletotrichum tofieldiae* selected form deposit identification numbers CECT 20833, CECT 20834, CECT 20835 or CECT 20836.

24. Use of the microorganism *Colletotrichum tofieldiae* and/or filtrate media of said microorganism, for increasing the production of flowers, seeds and/or fruits in a plant.

25. Use of the microorganism *Colletotrichum tofieldiae* and/or filtrate media of said microorganism for increasing chlorophyll production

26. Use of the microorganism *Colletotrichum tofieldiae* and/or filtrate media of said microorganism for increasing flowers, seeds and/or fruits production in a plant which is under drought or limited irrigation conditions.

27. Primers identified by sequences SEQ ID NO: 1 and 2.

## Patentansprüche

1. Verfahren zum Erhöhen der Produktion von Blüten, Samen und/oder Früchten in einer Pflanze, umfassend den Schritt des Kontaktierens der Pflanze mit einer Zusammensetzung, wobei die Zusammensetzung den Mikroorganismus *Colletotrichum tofieldiae* und/oder Filtrate des Mikroorganismus umfasst.

2. Verfahren nach Anspruch 1, **dadurch gekennzeichnet, dass** der Mikroorganismus ein Stamm von *Colletotrichum tofieldiae,* ausgewählt aus den Hinterlegungsidentifikationsnummern CECT 20833, CECT 20834, CECT 20835 oder CECT 20836, ist.

3. Verfahren nach einem der Ansprüche 1 oder 2, **dadurch gekennzeichnet, dass** der Mikroorganismus in Form von Sporen, Hyphen, Myzelien oder Sklerotien vorliegt.

4. Verfahren nach einem der Ansprüche 1 bis 3, **dadurch gekennzeichnet, dass** das Kontaktieren die Anwendung der Zusammensetzung auf Samen umfasst.

5. Verfahren nach einem der Ansprüche 1 bis 3, **dadurch gekennzeichnet, dass** das Kontaktieren die Anwendung der Zusammensetzung auf oberirdische Teile der Pflanze umfasst.

6. Verfahren nach einem der Ansprüche 1 bis 3, **dadurch gekennzeichnet, dass** das Kontaktieren die Anwendung der Zusammensetzung auf Wurzeln, Zwiebeln oder Knollen der Pflanze umfasst.

7. Verfahren nach einem der Ansprüche 1 bis 3, **dadurch gekennzeichnet, dass** das Kontaktieren die Anwendung der Zusammensetzung auf ein Substrat, das dazu imstande ist, die Pflanze zu kultivieren, umfasst.

8. Verfahren nach einem der Ansprüche 1 bis 7, **dadurch gekennzeichnet, dass** die Zusammensetzung Mineralien, organische Materialien, organische Verbindungen, anorganische Verbindungen, flüssige Verdünnungsmittel, Alkohle, Ketone, Pflanzenöle, aliphatische Kohlenwasserstoffe, Ester, Dimethylsulfoxid, Acetonitril, Wasser, anionische Tenside, nichtionische Tenside, kationische Tenside, wasserlösliche Polymere, Polysaccharide, Konservierungsstoffe, Farbstoffe und/oder Stabilisierungsmittel umfasst.

9. Verfahren nach Anspruch 8, **dadurch gekennzeichnet, dass** die Mineralien ausgewählt sind aus der Gruppe bestehend aus Ton, Bentonit, Calcit und Kieselgur;
die organischen Materialien ausgewählt sind aus der Gruppe bestehend aus Maispulver und Nussschalenpulver;
wobei die organische Verbindung Urea ist;
die anorganischen Verbindungen ausgewählt sind aus der Gruppe bestehend aus Calciumcarbonat, Ammoniumsulfat; Siliconoxid und Magnesiumaluminumsilikat;
wobei die flüssigen Verdünnungsmittel ausgewählt sind aus der Gruppe bestehend aus aromatischen Kohlenwasserstoffen, aliphatischen Kohlenwasserstoffen, Alkohlen, Ketonen, Pflanzenölen, Estern, Dimethylsulfoxid, Acetonitril und Wasser;
die anionischen Tenside ausgewählt sind aus der Gruppe bestehend aus Salzen von Sulfatealkylestern, Alkylarylsulfonatsalzen, Dialkyl Sulfosuccinatsalzen, Salzen von Polyoxyethylenalkylaryletherphosphatestern, Polyoxyethylenlignosulfonatsalzen und Formaldehydpolykondensaten;
die nichtionischen Tenside ausgewählt sind aus der Gruppe bestehend aus Alkylarylpolyoxyethylenethern, Alkylpolyoxipropylenblockcopolymeren und Polyoxyethylenfettsäureestern;
die kationischen Tenside Alkyltrimethylammoniumsalze sind;
die Polymere wasserlöslicher Polyvinylalkohol oder Polyvinylpyrrolidon sind;
die Polysaccharide ausgewählt sind aus der Gruppe bestehend aus Agar, Acacia, Alginsäure, Salzen von Alginsäure, Carboxymethylcellulose und Xanthangummi;
und die Stabilisierungsmittel Isopropylphosphatsäure oder BHT sind.

10. Verfahren nach Anspruch 9, **dadurch gekennzeichnet, dass** die aromatischen Kohlenwasserstoffe ausgewählt sind aus der Gruppe bestehend aus Xylen, Alkylbenzen und Methylnaphthalen;
die Alkohole ausgewählt sind aus der Gruppe bestehend aus 2-5-Propanol, Ethylenglycol, Propylenglycol und Ethylenglycolmonoethylether;
die Ketone ausgewählt sind aus der Gruppe bestehend aus Aceton, Cyclohexanon und Isophoron;
und die Pflanzenöle Sojaöl oder Baumwollsamenöl sind.

11. Verfahren nach einem der Ansprüche 1 bis 10, **dadurch gekennzeichnet, dass** die Zusammensetzung Substratfertigationsalze, Düngemittel, Insektizide, Nematizide, Fungizide, Bakterizide und/oder Herbizide umfasst.

12. Verfahren nach einem der Ansprüche 1 bis 11, **dadurch gekennzeichnet, dass** die Zusammensetzung eine Flüssigkeit, ein Feststoff, eine Paste oder ein Gel ist.

13. Verfahren nach einem der Ansprüche 1 bis 12, **dadurch gekennzeichnet, dass** die Zusammensetzung ein Pulver, eine Pille, eine Tablette, eine Flüssigkeit, ein Granulat oder ein emulgierbares Konzentrat ist.

14. Verfahren nach einem der Ansprüche 1 bis 13, **dadurch gekennzeichnet, dass** das Kontaktieren Anwendung durch Sprühen, Pulverisieren, Eintauchen, Bewässern oder Bestäuben umfasst.

15. Verfahren nach einem der Ansprüche 1 bis 14, **dadurch gekennzeichnet, dass** die Pflanze eine nacktsamige Pflanze oder eine zweikeimblättrige Pflanze ist.

16. Verfahren nach einem der Ansprüche 1 bis 14, **dadurch gekennzeichnet, dass** die Pflanze zu der Familie der *Brassicaceae* gehört.

17. Verfahren nach einem der Ansprüche 1 bis 14, **dadurch gekennzeichnet, dass** die Pflanze eine einkeimblättrige Pflanze ist.

18. Verfahren nach einem der Ansprüche 1 bis 14, **dadurch gekennzeichnet, dass** die Pflanze Gerste *(Hordeum vulgare)* ist.

19. Verfahren nach einem der Ansprüche 1 bis 14, **dadurch gekennzeichnet, dass** die Pflanze Mais *(Zea mays)* ist.

20. Pflanzenvermehrungsmaterial, das einen Stamm von *Colletotrichum tofieldiae,* ausgewählt aus den Hinterlegungsidentifikationsnummern CECT 20833, CECT 20834, CECT 20835 oder CECT 20836, umfasst.

21. Pflanzenvermehrungsmaterial nach Anspruch 20, wobei das Material ausgewählt ist aus der Gruppe bestehend aus Samen, Setzlingen, jungen Pflanzen, Ablegern, Zwiebeln und Knollen.

22. Substrat zur Pflanzenzüchtung, das den Mikroorganismus *Colletotrichum tofieldiae* und/oder die Mikroorganismusfiltrate umfasst.

23. Stamm des Mikroorganismus *Colletotrichum tofieldiae,* ausgewählt aus den Hinterlegungsidentifikationsnummern CECT 20833, CECT 20834, CECT 20835 oder CECT 20836.

24. Verwendung des Mikroorganismus *Colletotrichum tofieldiae* und/oder von Filtratmedien des Mikroorganismus zum Erhöhen der Produktion von Blüten, Samen und/oder Früchten in einer Pflanze.

25. Verwendung des Mikroorganismus *Colletotrichum tofieldiae* und/oder von Filtratmedien des Mikroorganismus zum Erhöhen der Chlorophyllproduktion.

26. Verwendung des Mikroorganismus *Colletotrichum tofieldiae* und/oder von Filtratmedien des Mikroorganismus zum Erhöhen der Produktion von Blüten, Samen und/oder Früchten Produktion in einer Pflanze, die Trockenheit oder begrenzten Bewässerungsbedingungen ausgesetzt ist.

27. Primer, identifiziert durch die Sequenzen SEQ ID NO: 1 und 2.

## Revendications

1. Procédé destiné à augmenter la production de fleurs, de graines et/ou de fruits dans une plante, comprenant l'étape de mise en contact de ladite plante avec une composition, la composition comprenant le microorganisme *Colletotrichum tofieldiae* et/ou des filtrats dudit microorganisme.

2. Procédé selon la revendication 1, **caractérisé en ce que** ledit microorganisme est une souche de *Colletotrichum tofieldiae* sélectionnée parmi les numéros d'identification de dépôt CECT 20833, CECT 20834, CECT 20835 ou CECT 20836.

3. Procédé selon l'une quelconque des revendications 1 ou 2, **caractérisé en ce que** ledit microorganisme se présente sous la forme de spores, d'hyphes, de mycélium ou de sclérotes.

4. Procédé selon l'une quelconque des revendications 1 à 3, **caractérisé en ce que** ladite mise en contact comprend l'application de la composition à des graines.

5. Procédé selon l'une quelconque des revendications 1 à 3, **caractérisé en ce que** ladite mise en contact comprend l'application de la composition sur des parties aériennes de la plante.

6. Procédé selon l'une quelconque des revendications 1 à 3, **caractérisé en ce que** ladite mise en contact comprend l'application de la composition aux racines, bulbes ou tubercules de la plante.

7. Procédé selon l'une quelconque des revendications 1 à 3, **caractérisé en ce que** ladite mise en contact comprend l'application de la composition sur un substrat apte à cultiver ladite plante.

8. Procédé selon l'une quelconque des revendications 1 à 7, **caractérisé en ce que** ladite composition comprend des minéraux, des matières organiques, des composés organiques, des composés inorganiques, des diluants liquides, des alcools, des cétones, des huiles végétales, des hydrocarbures aliphatiques, des esters, du sulfoxyde de diméthyle, de l'acétonitrile, de l'eau, des tensioactifs anioniques, des tensioactifs non ioniques, des tensioactifs cationiques, des polymères hydrosolubles, des polysaccharides, des conservateurs, des agents colorants et/ou des agents stabilisants.

9. Procédé selon la revendication 8, **caractérisé en ce que** lesdits minéraux sont choisis dans le groupe contistué de l'argile, la bentonite, la calcite et les épis de diatomées ;
les matières organiques sont choisies dans le groupe constitué de la poudre de maïs et de la poudre de peau de noix ;
ledit composé organique est l'urée ;
lesdits composés inorganiques sont choisis dans le groupe constitué du carbonate de calcium, du sulfate d'ammonium, de l'oxyde de silicium et du silicate de magnésium et d'aluminium ;
lesdits diluants liquides sont choisis dans le groupe constitué des hydrocarbures aromatiques, des hydrocarbures aliphatiques, des alcools, des cétones, des huiles végétales, des esters, du sulfoxyde de diméthyle, de l'acétonitrile et de l'eau ;
lesdits tensioactifs anioniques sont choisis dans le groupe constitué des sels d'esters alkyliques de sulfate, des sels de sulfonate d'alkylaryle, des sels de sulfosuccinate de dialkyle, des sels d'esters de phosphate d'éther de polyoxyéthylène alkylaryliques, des sels de lignosulfonate de polyoxyéthylène et des polycondensats de formaldéhyde ;
lesdits tensioactifs non ioniques sont choisis dans le groupe constitué d'éthers de polyoxyéthylène alkylaryliques, de copolymères séquencés d'alkylpolyoxypropylène et d'esters d'acides gras polyoxyéthyléniques ;
lesdits tensioactifs cationiques sont des sels d'alkyltriméthylammonium ;
lesdits polymères sont l'alcool polyvinylique soluble dans l'eau ou la polyvinylpyrrolidone ;
lesdits polysaccharides sont choisis dans le groupe constitué de la gélose, de la gomme arabique, de l'acide alginique, des sels de l'acide alginique, de la carboxyméthylcellulose et de la gomme de xanthane ;
et lesdits agents stabilisants sont l'acide phosphatique d'isopropyle ou le BHT.

10. Procédé selon la revendication 9, **caractérisé en ce que** lesdits hydrocarbures aromatiques sont choisis dans le groupe constitué par le xylène, l'alkylbenzène et le méthylnaphtalène ;
lesdits alcools sont choisis dans le groupe constitué du 2-5 propanol, de l'éthylène glycol, du propylène glycol et de l'éther monoéthylique d'éthylène glycol ;
lesdites cétones sont choisies dans le groupe comprenant l'acétone, la cyclohexanone et l'isophorone ;
et lesdites huiles végétales sont l'huile de soja ou l'huile de graine de coton.

11. Procédé selon l'une quelconque des revendications 1 à 10, **caractérisé en ce que** ladite composition comprend des sels de fertirrigation de substrat, des engrais, des insecticides, des nématicides, des fongicides, des bactéricides et/ou des herbicides.

12. Procédé selon l'une quelconque des revendications 1 à 11, **caractérisé en ce que** ladite composition est un liquide, un solide, une pâte ou un gel.

13. Procédé selon l'une quelconque des revendications 1 à 12, **caractérisé en ce que** ladite composition est une poudre, une pilule, un comprimé, un granulé ou un concentré émulsifiable.

14. Procédé selon l'une quelconque des revendications 1 à 13, **caractérisé en ce que** ladite mise en contact comprend une application par vaporisation, pulvérisation, immersion, irrigation ou époussetage.

15. Procédé selon l'une quelconque des revendications 1 à 14, **caractérisé en ce que** ladite plante est un gymnosperme ou une dicotylédone.

16. Procédé selon l'une quelconque des revendications 1 à 14, **caractérisé en ce que** ladite plante est de la famille des *Brassicaceae.*

17. Procédé selon l'une quelconque des revendications 1 à 14, **caractérisé en ce que** ladite plante est une monocotylédone.

18. Procédé selon l'une quelconque des revendications 1 à 14, **caractérisé en ce que** ladite plante est l'orge *(Hordeum vulgare).*

19. Procédé selon l'une quelconque des revendications 1 à 14, **caractérisé en ce que** ladite plante est le maïs*(Zea mays).*

20. Matériel de multiplication de plantes comprenant une souche de *Colletotrichum tofieldiae* sélectionnée parmi les numéros d'identification de dépôt CECT 20833, CECT 20834, CECT 20835 ou CECT 20836.

21. Matériau de propagation de plantes selon la revendication 20, dans lequel ledit matériau est choisi dans le groupe constitué de graines, de plantules, de jeunes plantes, de boutures, de bulbes et de tubercules.

22. Substrat pour la culture de plantes comprenant le microorganisme *Colletotrichum tofieldiae* et/ou les filtrats dudit microorganisme.

23. Souche du microorganisme *Colletotrichum tofieldiae* choisie parmi les numéros d'identification de dépôt CECT 20833, CECT 20834, CECT 20835 ou CECT 20836.

24. Utilisation du microorganisme *Colletotrichum tofieldiae* et/ou de milieux filtrants dudit micro-organisme pour augmenter la production de fleurs, de graines et/ou de fruits dans une plante.

25. Utilisation du microorganisme *Colletotrichum tofieldiae* et/ou de milieux filtrants dudit microorganisme pour augmenter la production de chlorophylle.

26. Utilisation du microorganisme *Colletotrichum tofieldiae* et/ou de milieux filtrants dudit microorganisme pour augmenter la production de fleurs, de graines et/ou de fruits dans une plante dans des conditions de sécheresse ou d'irrigation limitée.

27. Amorces identifiées par les séquences SEQ ID No : 1 et 2.
